# EUROPEAN PATENT APPLICATION

(11) **EP 3 073 759 A1**
(43) Date of publication of application: **28.09.2016**
(21) Application number: 15160323.0
(22) Date of filing: 23.03.2015
(51) Int. Cl.: H04R 1/10, A61B 5/0476, F21V 21/084, F21L 4/00

(54) **EAR-MOUNTED DEVICE**

(71) Applicant: Freebit AS, 0208 Oslo (NO)
(72) Inventor: Sandanger, Vidar, 0208 Oslo (NO)
(74) Representative: Acapo AS

(57) **Abstract**

Ear-mounted apparatus are provided for secure and comfortable load bearing in support of optical, biosensor, and mobile communication functionalities. In particular, a load-bearing ear unit is provided which includes an anchor for stable attachment in an ear and a member extending from the anchor. The member has therein a rotational stabiliser adapted to provide stability in relevant planes for secure ear mounting. Also provided is an ear-mounted optical device, comprising the load-bearing ear unit and one of a camera, a light source, a virtual reality device, a IrDA-device, and a photo diode. Further provided is an ear-mounted wearable device, comprising the load-bearing ear unit and one or more biosensors. A particular such wearable device is an ear-mounted EAG device.

## Description

### BACKGROUND OF THE DISCLOSURE

The present disclosure relates in general to ear-mounted devices. Specifically, the present disclosure relates to apparatus for removable ear attachment to optical devices, head-mounted or head-sensing devices. More specifically, a load-bearing ear unit for secure and comfortable attachment of a forward extending member is provided. Also provided in various embodiments are ear-mounted camera, flash lights, Virtual Reality (VR) device, Infrared Data Association (IrDA) device, and Electroencephalography (EEG) device incorporated therein the load-bearing ear unit of this disclosure.

Various forward extending head mounted devices are well known, such as glasses and head attached flash lights.

Many solutions use an ear hook attachment wherein a member is placed between the pinna of the ear and the skull, preferably with a downward extending hook anterior to the ear. This is the typical case for glasses. References should also be made to US6637910 regarding an ear attached flashlight using an ear hook.

However ear hook solutions suffer from several problems such as instability due to rotational momentum caused by the relatively long arm. If this is balanced using a counterbalance the total weight increases and correspondingly the comfort decreases. Also a counterbalance will not improve matter regarding the stability in following head movement. Moreover an ear hook device will interfere with use of traditional glasses. Stabilisation using the nose will also interfere with use of glasses and can also be uncomfortable particularly for heavier devices such as glasses, VR-equipment and the like.

Some solutions such as head mounted flashlight use a headband. Typically a flashlight or torch is positioned on the forehead of a user and a battery pack conveniently is located at the back, thereby functioning as a counterbalance.

However to provide stability the headband has to be relatively tight and thus less comfortable. Such a positioning is also far from unobtrusive. The positioning for devices such as light sources are also problematic since the illumination is close to co-axial with the eyes of the wearer since illumination strongly reduces shadowing effects and contours resulting in flat images or "whiteout". The presence of a counterbalance also increases weight and discomfort.

### SUMMARY OF THE VARIOUS EMBODIMENTS

It is therefore an object of this disclosure to provide new and improved methodologies and designs to correct the shortcomings of the aforementioned ear-mounting devices.

Particularly, in accordance with this disclosure, there is provided, in one embodiment, a load bearing ear unit comprising an anchor for stable attachment in an ear, and a member extending from the anchor, wherein the anchor comprises a rotational stabiliser thereby stabilizing the member.

In another embodiment, the anchor further comprises a housing unit forming an attachment connecting the member to the anchor.

In yet another embodiment, the rotational stabiliser is adapted to stabilize the member in the sagittal plane.

In a further embodiment, the anchor comprises an arm adapted as a decremental curve corresponding to the antihelix of the ear. The decremental curve falls along an inner part of the antihelix and is partly positioned under the antitragus.

In another embodiment, the rotational stabiliser is an end that extends into a cavity defined by the antihelix and root of helix.

According to another embodiment, the rotational stabiliser is a protrusion adapted to engage with an end surface of fossa triangularis.

In accordance with yet another embodiment, the rotational stabiliser is adapted to stabilize the member in the transverse plane.

In a further embodiment, the rotational stabiliser is a member abutting a part of pinna.

In one embodiment, the rotational stabiliser is a tragus support adapted to abut incisura anterior.

In another embodiment, the rotational stabiliser is a sulcus support adapted to abut sulcus auriculae posterior.

According to a further embodiment, the rotational stabiliser is a temple support adapted to abut temple.

In accordance with a further embodiment, the member further comprises a connector for mechanical connectivity. In another embodiment, the connector is rotatably adjustable. In yet another embodiment, connector is a magnet.

In accordance with another embodiment, the member further comprises a connector for power connectivity. In accordance with yet another embodiment, the member further comprises a connector for data connectivity. In another embodiment, the connector is one of the group including an audio jack, an audio jack socket, a USB socket, and a USB plug.

In accordance of this disclosure, there is provided, in another embodiment, an ear-mounted optical device that comprises a load bearing ear unit. The load-bearing ear unit comprises an anchor for stable attachment in an ear and a member extending forward from the anchor, wherein the anchor comprises a rotational stabiliser thereby stabilizing the member.

According to alternative embodiments, the optical device is one of a flash light, a display unit, a VR device, an IrDA-device, a photo diode, and a camera, and glasses.

According to another embodiment, there is provided an ear-mounted EEG device, comprising a first anchor for stable attachment in an ear and a first member extending forward from the anchor, wherein the first anchor further comprises a first EEG electrode, and wherein the first member further comprises a temple support pad adapted to be a second EEG electrode.

In a further embodiment, the EEG device further comprises a second anchor and extended therefrom a second member. The second anchor comprises a third EEG electrode. In another embodiment, the second member comprises a second temple support pad adapted to be a fourth EEG electrode.

In another embodiment, the EEG device further comprises a nose support, adapted to connect the first and second members.

In accordance of this disclosure, there is provided, in a further embodiment, an ear-mounted device that comprises a load bearing ear unit and one or more sensors. The load-bearing ear unit comprises an anchor for stable attachment in an ear and a member extending from the anchor, wherein the anchor further comprises a sensor adapted to detect signals when the device is worn by a user, from a part of the user's ear.

In another embodiment, the member further comprises a sensor adapted to detect signals when the device is worn by a user, from a part of the user's head.

### BRIEF DESCRIPTION OF THE DRAWINGS

Fig. 1A shows the anatomy of a human ear from the outside.
Fig. 1B shows a cross section of the anatomy of a human ear along A - A.
Fig. 2A depicts an ear unit inserted into an ear according to one embodiment,
Fig. 2B depicts an ear unit inserted into an ear shown in an intersection along B - B according to one embodiment.
Fig. 2C depicts s an ear unit inserted into an ear showing an intersection along B - B according to one embodiment,
Figs. 3 and 4 depict a load-bearing ear unit according to one embodiment.
Figs. 5.1 and 5.2 depict two load-bearing ear units according to alternative embodiments.
Fig. 6 depicts a pair of glasses comprising two load-bearing ear units according to one embodiment.

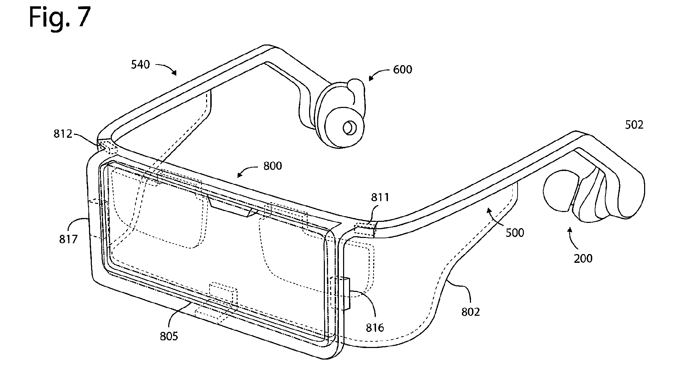

Fig. 7 depicts a carrier carrying therein a cell phone and connected to two load bearing arms according to one embodiment.

The following table set forth reference numbers and the corresponding annotation according to various embodiments as shown in the drawings.

| | |
|---|---|
| 10 | Pinna |
| 11 | Helix |
| 11a | Flap |
| 12 | Crus of helix |
| 13 | Antihelix |
| 14 | Crura of antihelix |
| 15 | Superior crux |
| 16 | Inferior crux |
| 17 | Fossa triangularis |
| 18 | Sulcus auriculae posterior |
| 19 | Incisura anterior |
| 21 | Tragus |
| 22 | Antitragus |
| 23 | Incisura intertragica, intertragic notch |
| 24 | Concha |
| 25 | Cymba concha |
| 26 | Cavum concha |
| 27 | Scapha |
| 28 | Sub-tragus region |
| 29 | Tympanic membrane, eardrum |
| 30 | Ear canal |
| 100 | Ear unit |
| 200 | Anchor |
| 210 | Curve |
| 212 | Upper end |
| 214 | Lower end |
| 216 | Extended lower end |
| 218 | Curvature |
| 220 | Protrusion |
| 222 | Protrusion end, for engaging fossa triangularis |
| 224 | Protrusion for engaging intertragic notch |
| 300 | Housing |
| 400 | Part extending downwards |
| 500 | Member, forward extending |
| 502 | Member attachment to housing or to anchor |
| 504 | Arm, extending forward |
| 506 | Tragus support |
| 508 | Sulcus support |
| 510 | Front, arm payload |
| 512 | Display unit |
| 516 | Temple support |
| 520 | Rear, counterbalance |
| 530 | Transverse arm |
| 532 | Nose support |
| 533 | Left connector between transverse arm and arm extending forward |
| 534 | Left part of transverse arm |
| 535 | Connector connecting left and right part of transverse arm |
| 536 | Right part of transverse arm |
| 537 | Right connector between transverse arm and second arm extending backward |
| 540 | Second arm, extending backward |
| 546 | Second temple support |
| 600 | Second audio device, second anchor |
| 602 | Wire |
| 700 | Rear unit |
| 705 | Rear arm |
| 706 | Light source |
| 800 | Carrier |
| 802 | Side shadow |
| 805 | Cell phone received into carrier |
| 811 | Connector for engaging connector on the arm extending forward |
| 812 | Connector for engaging connector on the second arm |
| 816 | Connector engaging a first connector on the cell phone |
| 817 | Connector engaging a second connector on the cell phone |

### DETAILED DESCRIPTION OF THE VARIOUS EMBODIMENTS

### Anatomy of the Human Ear

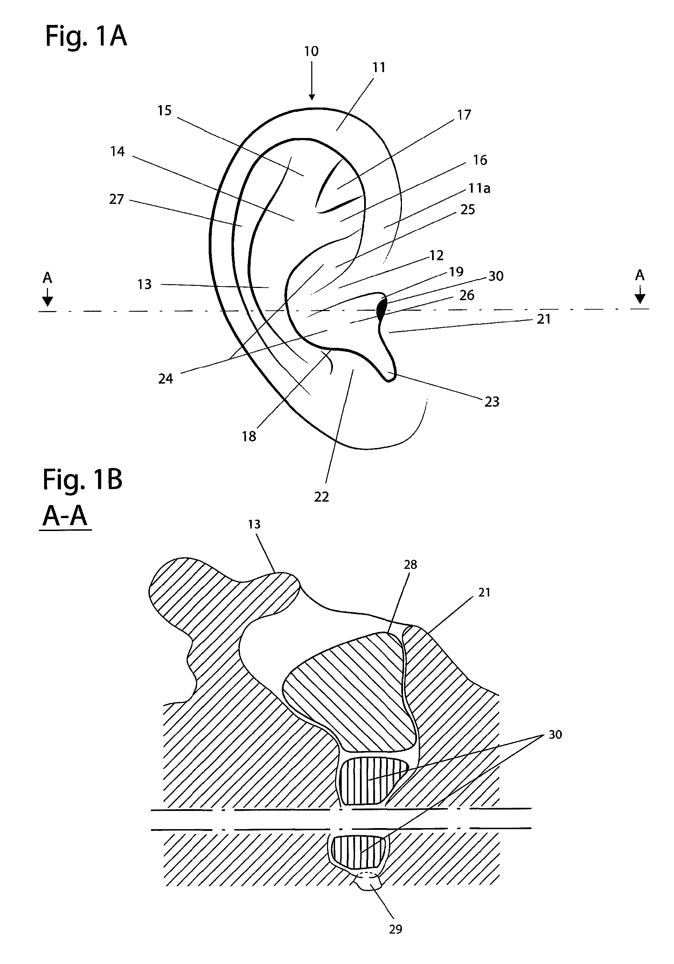

Referring to Figure 1A, the structure of a human ear, and in particular of the outer ear, is depicted. Various features of the outer ear as described herein are closely related to the various embodiments of the ear-mounted apparatus according to this disclosure.

Specifically, outer ear 10, also known as pinna comprises a plurality of features of significance. Outermost is helix 11 tracing the periphery of the ear upwards and in towards the skull where it transitions into crus of helix 12. Within this is antihelix 13 which in the upward direction bifurcates into crura of antihelix 14, comprising superior crux 15 and inferior crux 16, separated by fossa triangularis 17. Below antihelix is sulcus auriculae posterior 18 and further below that again is antitragus 22 which is opposite tragus 21, separated by incisura intertragica 23, also known as the intertragic notch. Within these again is concha 24 which comprises cymba concha 25 and cavum concha 26, separated by crus of helix 12.

It should be noted that a the part of helix near where it transitions into crus of helix forms a flap 11a that covers the anterior part of fossa triangularis and the anterior part of the upper part of the antihelix.

Immediately within and partially covered by the tragus is the entrance to the ear canal 30. It is important to realize that this entrance is still a part of cavum concha. The ear canal proper extends from the deepest part of the concha to the eardrum 29, a distance of about 2.5 cm and approximately 4 cm from the tragus. The ear canal comprises an approximately 8 mm lateral cartilaginous part and an approximately 16 mm medial osseous part. It should also be noted that it is typically quite uncomfortable to have any foreign object in the ear canal. The ear canal is partially visible from the outside and is indicated in Fig. 1A.

This area immediately within and partially covered by the tragus 21 does not have an official anatomical name. For the purposes of this disclosure it has been called the sub-tragus region 28 and is shown in Fig. 1B. The ear canal 30 is located below the sub-tragus region.

### Stability of Ear-Mounted Apparatus

A movable object in space can be described with 6 degrees of freedom: linear: anterior - posterior, dorsal - ventral, and left - right lateral; rotational: in the sagittal plane, in the coronal plane, and in the transverse plane.

For an ear mounted or head mounted device these axes and planes are defined with reference to the head according to anatomical terms of location. For a device to be stably positioned into an ear it is important that linear motion is restricted relative to the ear, otherwise the device will fall out. Similarly it is strongly preferred that rotational motion relative to the head is restricted. For use for optical devices flash light, display unit such as VR devices or camera such stability is important for the user experience. Also a rapid rotational movement of the head can also create a force that pulls the device out of the ear.

For a device having a member extending forward this means that a static rotational force in the sagittal plane is created as gravity pulls the member downwards. This then must be counteracted by a rotational stabiliser.

For a VR device stability means that the unit remains stable with respect to the head of the user. On the other hand stability for a camera means that the unit should be stable with reference to space to avoid shaking images. In practice this means that high frequency movements should be damped while still allowing the camera to track the general motion of the head.

### Load-Bearing Ear Unit

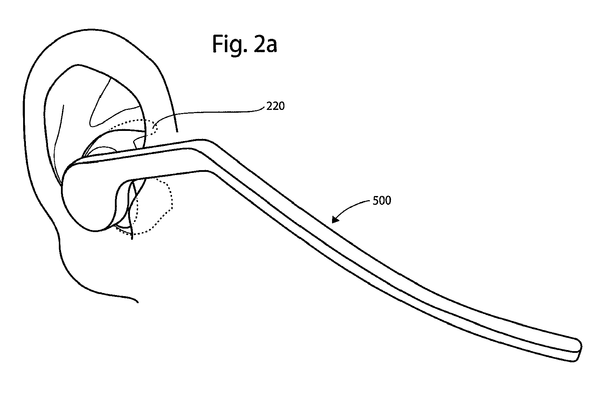

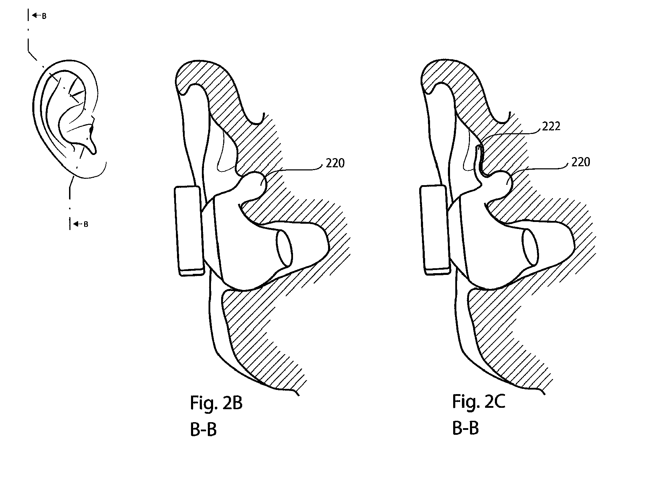

Referring to Fig. 2A, an ear unit fitting into an ear according to one embodiment of this disclosure is shown from the outside. A line A - A indicates a section for Fig. 2B and Fig. 2C. The anchor 200 comprises the curve 210 having an upper end 212, a lower end 214 and a curvature 218. Due to the angles it is not possible to clearly show all components of the anchor in all figures.

In this embodiment, an anchor 200 provides stable attachment in an ear, and a member extending forward from the anchor. The anchor is provided with a rotational stabiliser, and the member is stabilised in the sagittal plane and becomes vertically stable.

The rotational stabiliser presents several embodiments according to this disclosure. Of relevance, for example, the pinna 10 of the ear comprises several anatomical details that a part, particularly a protuberance, of an anchor can extend into to counteract a rotation in the sagittal plane by the fact that these effectively form cavities, sacks or trenches (Latin: «fossae») having an end surface that said protuberance can extend to in order to create a force counteracting the rotational momentum.

According to another embodiment, in addition to stability against rotation in the sagittal plane, stability in the transverse plane is provided.

Some of this stability is inherent in the anchor according to one embodiment, as it is stable against falling out. In a further embodiment, the member is adapted to contact parts of the head of the user. For compactness and comfort, in one embodiment, this contact is made against parts of the pinna 10.

While the sagittal plane and transversal plane stabilities are achieved by separate means it should be noted that the use of supports for transversal plane stability also improves sagittal plane stability according to alternative embodiments.

### Various Embodiments of Ear-Mounted Apparatus

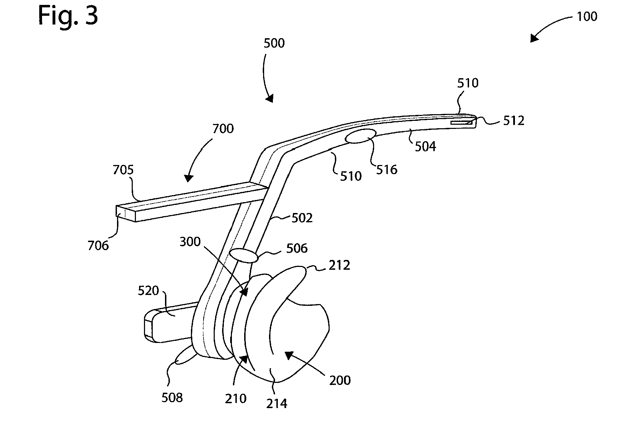

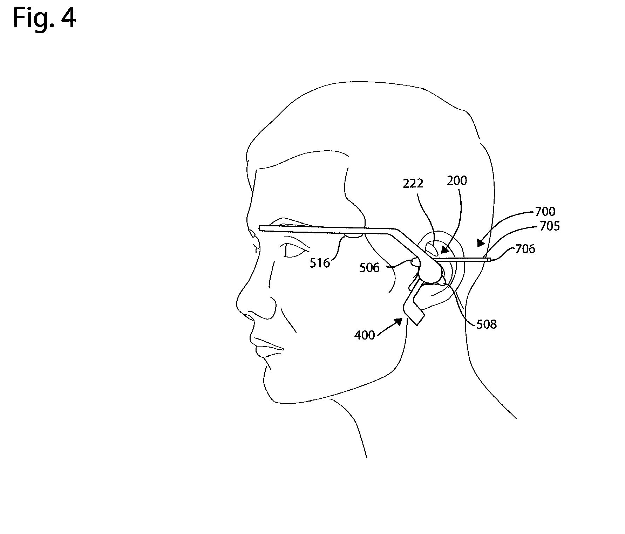

Referring to Figures 3 and 4, a load bearing ear unit as one embodiment of the ear-mounted device according to this disclosure is provided. This ear unit comprises an anchor adapted as the C-shape according to the applicant's prior patents and applications, specifically as described in WO/2002/045390, and as the improved earpiece having a curve 210 and a curvature 218 as described in WO/2008/147215, the curve having an upper end 212 and a lower end 214.

In one embodiment, the anchor is based on the C-shape known from WO/2002/045390 or the curve known from WO/2008/147215, both disclosed and taught by the present Applicant. The latter comprises a curvature that provides some stabilisation.

In another embodiment the anchor 200 comprises a curve 210 having an upper end 210 that extends into an upper anterior part of the antihelix forming a cavity defined by the antihelix and the root of the helix. According to this embodiment, the upper end is adapted to abut or engage an end surface of the upper or dorsal part of the antihelix. The root of the helix forms an end surface that the upper end of the curve engages to in order to create a force counteracting the rotational momentum in this embodiment. Accordingly, as the concha is close to the skull, the force can be transferred over a short distance and with limited elasticity and thereby providing improved stability. These anchors are stable with respect to linear motion and thus do not easily fall out of the ear.

According to another embodiment, for rotational stability in the transverse plane the member 500 is provided with supports 506, 508. The tragus support 506 is adapted to engage with incisura anterior 19 located between the upper part of tragus and a lower part of the anterior part of helix. Since this anatomical detail forms a depression into which a support pad fits snugly this provides rotational stability in both the transverse plane and the sagittal plane. The sulcus support 508 is adapted to engage with sulcus auriculae posterior 18 located posterior to the anti tragus. Since this anatomical detail forms a plateau it will provide limited stability in the transverse plane beyond what an anchor according to the above three documents but will represent an improvement over simpler anchors like earbuds.

In another embodiment the ear unit 100 comprises a housing 300 attached to the anchor 200. The housing unit is suitable for containing electronics and a power source. Also the housing unit is useful for containing acoustic devices for providing the user with an audio experience. Such a combination overcomes a common design problem with VR glasses that attach using traditional ear hooks and also contain earbuds that have to be individually adjusted and attached to the ear or ears. In this embodiment the concha is used for attachment and audio and thus no separate audio devices are required, leading to a more compact and convenient design.

In a further embodiment the housing unit comprises a sub tragus audio device as disclosed in WO/2014/017922, from the present applicant.

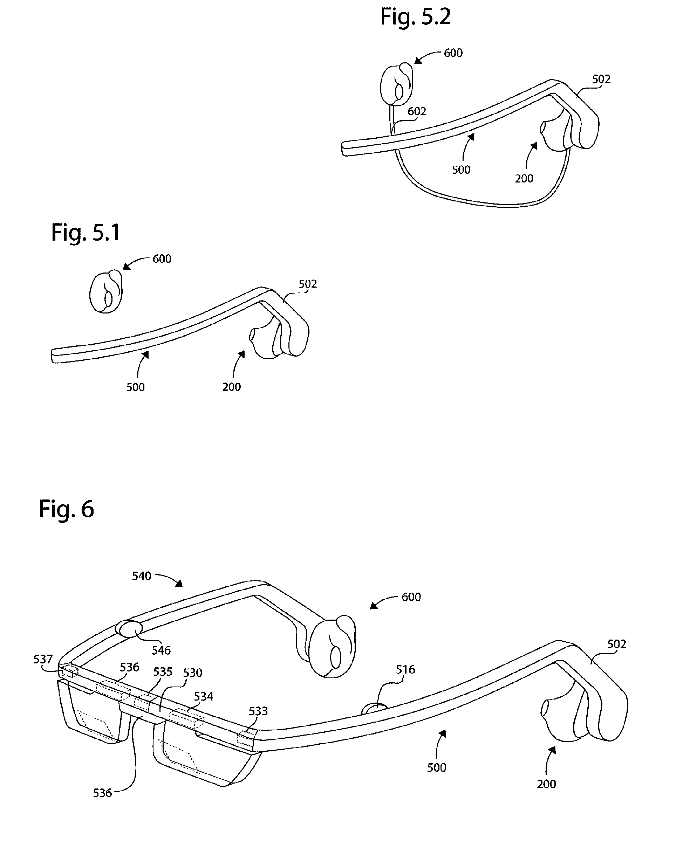

Referring to Fig. 5.1, in another embodiment, the audio device operates together with a second audio device 600 to create a stereo sound experience. In an alternative embodiment, this second audio device is connected to a common electronic unit in the housing, either wirelessly or as shown in Fig. 5.2 using a wire 602.

In one embodiment, the member 500 comprises an attachment 502 to the housing 300. From the attachment an arm 504 extends forward or anterior to the user. The arm is preferably thin and light weight both for stability reasons as well as for aesthetic reasons according to this embodiment.

According to another embodiment, the attachment 502 also provides an offset and is at an angle with respect to the arm 504. Using an offset it is possible to put on or remove a pair of glasses while keeping the ear unit inserted.

In certain embodiments, the front 510 of the arm is provided with the relevant payload. In another embodiment, the payload is a device 512 for overlaying an image to the view field of the user using a projector that projects an image onto the eye or via a glass.

To avoid interference with the use of glasses, in one embodiment the member extends upwards and forward so that the front 510 is positioned above the upper edge of the glasses.

According to another embodiment, the load bearing ear unit is adjustable to the use by different individual users. In one embodiment the member is rotatable with respect to the stabiliser against rotation in the sagittal plane. This is achieved in this embodiment by adjusting the length of the upper end of the curve.

In another embodiment, the attachment to the housing 502 and/or the arm 504 is adjusted lengthwise, for instance by a telescoping structure.

According to one embodiment, the arm is rotatably adjusted relative to the housing. Such rotation is used to raise the arm 500 in this embodiment carrying virtual reality glasses, out of sight. For adjustment when in active use, according to a certain embodiment, the rotation function is provided with a ratcheting function or a lock thereby holding the arm at desired angle.

Referring to Fig. 4, in a further embodiment a part 400 is provided that extends downwards. According to this embodiment, the part 400 contains electronics, battery, power sources or other parts that has substantial mass. In a further embodiment, for wired devices the wire is attached to this downward extending part.

According to another embodiment, this downward extending part extends along the intertragic notch 23. According to an alternative embodiment, this part also passes through the intertragic notch thereby providing additional stability. In another embodiment, this part is detachably attached to the ear unit.

According to a further embodiment, this part curves in the posterior direction thereby balancing the forces from the arm 500. In another embodiment, this part takes on in part the functionalities of the rear unit 700.

In a particular embodiment of this disclosure, the above configuration is utilized for reading out brain waves in a manner similar to electroencephalography (EEG). It is noted that prior systems such as WO2011006681 have the advantage of reading out brain signals in the ear canal in a compact solution but also the disadvantage of being able to read out only in one location and also in a situation subjected to noise from muscular activities. The ear-mounted EEG device according to one embodiment of this disclosure overcomes those limitations by complementing an in-ear sensor with a sensor integrated with temple support 516 and/or 546.

Using substantially rigid arms positioned using ear canals or the entrance to the ear canals as a reference point, provides the system with a stable geometry that is beneficial for reliability and also reproducibility between measurements. This overcomes a manual and error prone process in prior art where electrodes are carefully placed on the scalp of a subject to be analysed.

In another embodiment a nose support 320 is used to further improve positional stability and/or reproducibility of the electrodes.

In yet another embodiment, muscular activities are detected by means provided with the ear mounted system. Several such means are employed in various embodiments and can be freely combined to ensure misinterpretations are kept to a minimum. A camera records eye movements in one embodiment, where eye movements imply muscle movements leading to noise. A microphone records the user speaking in another embodiment, itself a strong indicator of muscle movements. In another embodiment, a mechanical sensor provided with the temple support detects relative motion in the skin of the user, indicating muscle motions in the head.

### Further Embodiments of Ear-Mounted Apparatus

In an alternative embodiment, the stabiliser is a protrusion 220 extending from the anchor or the housing and having an end 222 adapted to engage or abut an end surface of the fossa triangularis 17. Note that the protrusion according to this embodiment achieves rotational stability; it does not provide attachment to the ear. As fossa triangularis is a more peripheral part of pinna than concha, the counteracting force in this embodiment, therefore, is transmitted across an extended part of tissue compared with the antihelix stabiliser.

According to another embodiment, the above stabiliser is combined with the antihelix stabiliser discussed herein earlier. In a further embodiment, these stabilizers are blended into a single stabiliser. The end wall in both embodiments is defined by the helix. As a result, the pressure is exerted over a larger area thus improving comfort.

Further, rotational stability in the transverse plane are achieved by various alternative embodiments. In one embodiment the arm is provided with support in the form of a temple support 516 that contacts the temple anterior to the ear for stability. Since the temporal bone underneath the temple is not connected to the jawbone, speaking will not cause the member to move.

In another embodiment, for further improved stability the ear unit is provided with a member or protrusion 224 entering and engaging the intertragic notch 23. In an alternative embodiument, the protrusion 224 engaging the intertragic notch is the sole rotational stabiliser.

In a further embodiment, the ear unit comprises a counterbalance 520 at the rear of the of the ear unit. This is beneficial for particularly large payloads at the front 510 of the arm. In one embodiment, the counterbalance is detachably attached to the ear unit. In another embodiment, the counterbalance contains a power source such as a battery.

In an alternative embodiment, the member extends forward such that the front 510 is positioned to the side of the edge of the glasses, thereby avoiding interference with the use of glasses.

In a further embodiment, an ear-mounted optical device according to this disclosure comprises the load-bearing ear unit and a light source, a camera, and combination thereof. In various embodiments, the lighting and camera comprises infrared functionality such as infrared communication, using for example IrDA, relay function and wireless communication.

According to another embodiment, the load-bearing ear unit further comprises a rear unit 700 having a rear arm 705. In a further embodiment, this ear unit further comprises a light source 706. Accordingly, together with a light source at the front 510 of the member 500 the device can be used by pedestrian and cyclists for illumination of the ground as well as for indication to others in traffic.

In yet another embodiment the member 500 and/or the rear unit 700 are detachably attached to the ear unit, optionally replaced with units having different functionality such as added battery capacity, camera or a microphone arm.

Referring to Fig. 6, in another embodiment, the member 500 is provided with a transverse arm 530, preferably extending from the front 510 of the member 500 extending forward. In this embodiment, this transverse arm supports a glass 532. This glass is used to overlay images onto the view field of the wearer by projection according to this embodiument. In another embodiment, the glass is adapted to correct vision. In a further embodiment, the transverse arm is extended to provide support for a second glass. In another embodiment, further stability is provided by nose-pieces. The nose pieces according to this embodiment stabilize the apparatus with respect to the head of the user thereby providing a comfortable viewing experience. Moreover in another embodiment the transverse arm is connected to a second arm 540 extending backward towards the second ear and is further provided with a second temple support.

In alternative embodiments the transverse arm is segmented and functionally comprises at least part of the glasses such as for rimless designs. For instance a binocular set of glasses can be separated into a left and/or right monocular glass.

In such segmentation the transverse arm of this embodiment carrys binocular glasses and are divided into a left 534 and a right 536 part. These are detachably attached and are connected using a connector 535 in another embodiment.

In another embodiment the transverse arm is used to contain a connecting wire between two ear units, in a manner similar to wire 602.

According to one embodiment, several optionally detachable parts 400, 500, 520, 700 are provided in the load-bearing ear unit. These detachable parts are used in another embodiment to change the mechanical response to movement of the head, for example the change between the aforementioned stability preferred for VR use and stability preferred for camera use. Such response is modified according to alternative embodiments using weights as well as varying spring properties of the member and/or rear unit. Each of the parts is provided with a range of masses to achieve the desired response in various embodiments.

In another embodiment, detachable parts of different sizes and lengths are provided for adaption to users with different head sizes. These parts are made extensible according to one embodiment, like telescopic rods. The apparatus in one embodiment is relatively insensitive to dimensions for applications such as light sources or camera. For VR applications in another embodiment, the forward extending member is adapted far enough forward to provide an image in focus and positioned with respect to the eyes. In this embodiment, the member 500 is rotated with respect to the ear or the member 502 is adapted accordingly.

According to one embodiment, when using a left and right ear unit together it is advantageous to provide the front of the arm 510 with different payloads such as a flash on a first ear unit and a camera on a second ear unit. The offset created between the two ear units avoids the red eye effect that is observed when camera and flash are close together. Also this configuration provides better illumination and shadows, avoiding whiteout.

In another embodiment the payload is detachably attached to the front 510 of the member 500 extending forward. This way the payload can be changed, replaced or upgraded, for instance between camera, microphone, light source and other optical, communication, wearable devices.

In a further embodiment the attachment comprises a plug, for instance a USB plug, Firewire plug, providing power and/or data connectivity as well as mechanical connectivity. In another embodiment, the mechanical connectivity is further augmented by magnets, mechanical attachments or similar. These attachments are rotatably adjusted to accommodate a wider range of payloads in various embodiments.

The ear-mounted device according to various embodiments are applied in video viewing, virtual reality, and augmented reality displays. Such displays are monocular or binocular according to different embodiments. In the latter case according to a further embodiment, the binocular display employ a second arm 540 comprising further mechanical, power and data connectivity.

For connectivity involving two arms 504, 540 bridged electrically by a transverse arm 530, in another embodiment a wire 603 is replaced by the connectivity provided by this transverse arm.

Connectivity according to an alternative embodiment comprises detachable attachment between arm 504 and transverse arm 530 using a connector 533. The connection is an electrical connector such as USB in one embodiment. Similarly the second arm 540 is detachably attached to transverse arm 530 using a second connector 537 according to another embodiment. This is combined with the use of connector 535 for a split transverse arm according to a further embodiment. Thus full flexibility regarding payload, also asymmetric payload, are achieved according to various embodiments.

Connectivity is asymmetric according to certain embodiments. In one embodiment, the first arm 500 is connected using USB to a first end of a cell phone and the second arm 540 is connected using an audio jack to a second end of said cell phone. According to another embodiment, a cell phone is provided with a USB socket at the lower end and an audio socket at the upper end. According to yet another embodiment, the cell phone is connected to the load bearing unit by a transverse arm as described above.

The load bearing system provides multiple points of attachment according to various embodiments. For instance in addition to being in electrical contact with a cell phone as described above, according to another embodiment, the load bearing system provides attachment for lenses positioned between the cell phone display and the eyes of the wearer.

In alternative embodiments, each anchor is provided with a plurality of arms. In one embodiment, one arm is used for a display that is kept steady with respect to the head whereas the second arm is provided with a camera that is kept steady with respect to space. This is done using varying degree of stabilisation and/or damping with respect to the head. Thus a camera is more loosely coupled, with limited damping and preferably no strong stabilisers such as a temple support in this embodiment. As mass of camera and activity of user varies, in various embodiments varying damping is applied with respect to the anchor.

In another embodiment, the payload is carrier 800 for placing a display. In a further embodiment, the display is a cell phone 805. In one embodiment, the carrier is attached to at least one arm 504, 540 and is adapted to receive a display or a cell phone. In another embodiment, the carrier is provided with connectors 816, 817 that attach to the cell phone, either using flexible wire or more preferably using connectors that are located in positions to directly engage with the cell phone. These connectors are connected via connectors 811, 812 to the arms in one embodiment. The carrier is adapted to specific units, displays or cell phones in different embodiments.

In a further embodiment, one or two side shadows 502 is employed to prevent side glare from interfering with the viewing. In another embodiment the side shadows are detachable attached to the arms 504, 540 or the carrier 800.

In another embodiment a part of the part extending forward is used for payload. Such payload comprises electronics and batteries in one embodiment. In a further embodiment, the part extending forward is used for antennae.

In summary, according to various embodiments, the ear-mounted apparatus of this disclosure provides secure and comfortable load bearing in support of optical, biosensor, and mobile communication functionalities. The ear-mounted device disclosed herein avoids bulky counterbalances as part of the system; uses light weight and compact power sources; corrects instability due to rotational momentum; supports small and inconspicuous devices; allows wearing for extended period of time; allows for offsetting and thus improved illumination; and provides for integration with other in-ear and head-mounted devices.

The descriptions of the various embodiments provided in this disclosure, including the various figures and examples, are to exemplify and not to limit the invention and the various embodiments thereof.

## Claims

1. A load bearing ear unit (100), comprising an anchor (200) for stable attachment in an ear, and a member (500) extending from the anchor, wherein said anchor comprises a rotational stabiliser (210, 220, 224, 506, 508, 510), thereby stabilizing said member.

2. The load bearing ear unit of claim 1, further comprising a housing unit (300) forming an attachment connecting said member (500) to said anchor (200).

3. The load bearing ear unit of claim 1, wherein said rotational stabiliser is adapted to stabilize said member in the sagittal plane.

4. The load bearing ear unit of claim 1, wherein said anchor comprises an arm adapted as a decremental curve corresponding to the antihelix of the ear, wherein said decremental curve falls along an inner part of the antihelix and is partly positioned under the antitragus.

5. The load bearing ear unit of claim 4, wherein said rotational stabiliser is an end (212) that extends into a cavity formed between the antihelix and root of helix.

6. The load bearing ear unit of claim 3, wherein said rotational stabiliser is a protrusion (220) having an end (222) adapted to engage with an end surface of fossa triangularis (17).

7. The load bearing ear unit of claim 3, wherein said rotational stabiliser is a protrusion (224) adapted to abut intertragic notch (23).

8. The load bearing ear unit of claim 1, wherein said rotational stabiliser is adapted to stabilize said member in the transverse plane.

9. The load bearing ear unit of claim 8, wherein said rotational stabiliser is an extra member abutting a part of pinna (10).

10. The load bearing ear unit of claim 8, wherein said rotational stabiliser is a tragus support (506) adapted to abut incisura anterior (19).

11. The load bearing ear unit of claim 8, wherein said rotational stabiliser is a sulcus support (508) adapted to abut sulcus auriculae posterior (18).

12. The load bearing ear unit of claim 8, wherein said rotational stabiliser is a temple support (510) adapted to abut temple.

13. An ear-mounted optical device, comprising: i) a load bearing ear unit, and ii) one of the group including flash light, display unit, VR device, IrDA-device, photo diode, and camera, wherein said load-bearing ear unit comprises an anchor for stable attachment in an ear and a member extending from the anchor, and wherein said anchor comprises a rotational stabiliser thereby stabilizing said member.

14. The load bearing unit (100) of claim 1, wherein said member (500) further comprises a connector for mechanical connectivity.

15. The load bearing unit (100) of claim 14, wherein said connector is rotatably adjustable.

16. The load bearing unit (100) of claim 14, wherein said connector is a magnet.

17. The load bearing unit (100) of claim 1, wherein said member (500) further comprises a connector for power connectivity.

18. The load bearing unit (100) of claim 1, wherein said member (500) further comprises a connector for data connectivity.

19. The load bearing unit of claim 17 or 18, wherein said connector is one of the group including an audio jack, an audio jack socket, a USB socket, and a USB plug.

20. An ear-mounted EEG device (100), comprising a first anchor for stable attachment in an ear and a first member extending forward from the anchor, wherein the first anchor further comprises a first EEG electrode, and wherein the first member further comprises a temple support adapted to be a second EEG electrode.

21. An ear-mounted EEG device of claim 20, further comprising a second anchor and extended therefrom a second member, wherein said second anchor comprises a third EEG electrode.

22. An ear-mounted EEG device of claim 21, wherein said second member comprises a second temple support adapted to be a fourth EEG electrode.

23. An ear-mounted EEG device of claim 22, further comprising a nose support, adapted to connect the first and second members.

24. An ear-mounted device comprising a load bearing ear unit and a sensor, wherein said load bearing ear unit comprises an anchor for stable attachment in an ear and a member extending from said anchor, and wherein said anchor further comprises a sensor adapted to detect signals when said ear-mounted device is worn by a user, from a part of the user's ear.

25. A ear-mounted device of claim 24, wherein said member further comprises a second sensor adapted to detect signals when said ear-mounted device is worn by a user, from a part of the user's head.
